# EUROPEAN PATENT SPECIFICATION

(11) **EP 4 291 094 B1**
(45) Date of publication and mention of the grant of the patent: **29.04.2026**
(21) Application number: 22704908.7
(22) Date of filing: 08.02.2022
(51) Int. Cl.: A61B 5/35

(54) **COMPUTER DEVICE FOR REAL-TIME ANALYSIS OF ELECTROGRAMS**
RECHNERVORRICHTUNG ZUR ECHTZEITANALYSE VON ELEKTROGRAMMEN
DISPOSITIF INFORMATIQUE D'ANALYSE EN TEMPS RÉEL D'ÉLECTROGRAMMES

(30) Priority: 09.02.2021 FR 2101234
(43) Date of publication of application: 20.12.2023
(73) Proprietor: Substrate HD, 13008 Marseille (FR)
(72) Inventor: MOHR DURDEZ, Theophile, 13008 MARSEILLE (FR)
(74) Representative: Plasseraud IP
(86) International application number: PCT/EP2022/053048
(87) International publication number: WO 2022/171638

(56) References cited:
- EP-A1- 3 513 710
- EP-A1- 3 616 605
- EP-A2- 3 744 282
- WO-A1-2018/119316
- FR-A1- 3 079 405
- CAI WENJUAN ET AL: "Accurate detection of atrial fibrillation from 12-lead ECG using deep neural network", COMPUTERS IN BIOLOGY AND MEDICINE, NEW YORK, NY, US, vol. 116, 2 August 2019 (2019-08-02), XP086008913, ISSN: 0010-4825, [retrieved on 20190802], DOI: 10.1016/J.COMPBIOMED.2019.103378

## Description

The invention concerns a computer device for real-time analysis of electrograms.

The field of atrial fibrillation has been rapidly developing in the past 5 years.

The detection of cardiac areas that promote atrial fibrillation includes both off-line computing devices using software such as Topera and CardioInsight, and real-time computing devices using software such as CARTO.

The Topera software aims to reconstruct the electrical activation of the atria of the heart. Signal acquisition is carried out using a "basket catheter" (a catheter that is deployed over the entire atrium). The analysis is performed in delayed time, more than 2 minutes after the start of the acquisition. This solution is inconvenient because this type of catheter is difficult to insert and because the contact of the electrodes is not guaranteed. The analysis duration is very long, and the reconstruction does not allow high-definition maps to be obtained, and even fails in the case of complex electrical activation (which represents 70% of atrial fibrillation cases) due to an overly simplistic reconstruction.

The CardioInsight software aims to reconstruct the electrical activity of the heart using a multi-electrode electrocardiogram measurement vest on the patient's skin. The patient wears the vest before the operation, the data is analyzed and accessible during the operation. The analysis is done in delayed time (more than 15 minutes) after the beginning of the acquisition. This solution has the disadvantage of a very long computation time, which imposes a deferred time making it difficult to set up (the patient is required to come a few days ahead and wear the vest so that the data can be extracted before the operation), and a very high cost. In addition, the reconstruction does not allow high-definition maps because the measurements are set up too far apart, and fails in the case of complex electrical activation because of an overly simplistic reconstruction.

The CARTO software published by Biosense & Webster allows the implementation of algorithms to detect cardiac arrhythmias.

The CFAE (Complex Fractionated Atrial Electrograms) algorithm calculates the number of deflection points (variation of the sign of the derivative) in the signals and produces color maps in real time. The practitioner then interprets these complex maps to determine places of interest. This algorithm is not very specific and relatively simple.

The Ensite software published by Abbott Laboratories allows the implementation of algorithms to detect cardiac arrhythmias.

The Ripple algorithm (Ripple Mapping) is a module of the CARTO software that allows to reproduce the electrical activity of the atria after a first catheter passage. Both the amplitude and the propagation of electrical waves are made visible through this module. This results in maps that are extremely complex for the practitioner to understand. While this may work in simple cases, the analysis fails in the case of complex electrical activation because the maps are too difficult to interpret.

The Applicant invented an improved system and filed a French patent application FR1852850 which disclosed using two interlinked methods in order to perform real-time detection of cardiac areas that promote atrial fibrillation. More specifically, this invention discloses using a classifier arranged to apply two classification models to electrogram data. One model is faster to apply and less precise than the other, such that it is used to alert the practitioner of the potential interest of a cardiac region.

The further works of the Applicant have allowed to determine an improved computer device for real-time detection of cardiac areas promoting atrial fibrillations comprising a memory arranged to receive real-time electrograms signals each originating from one of a plurality of electrodes, a first evaluator comprising an extractor and a gradient boosting based machine learning module, said extractor being arranged to extract a set of features comprising at least one timewise analysis feature and at least one morphological feature from each electrogram signal within a set of electrogram signals, and to feed the resulting sets of features to said gradient boosting based machine learning module trained on data comprising sets of features labelled with a value indicating whether the associated electrogram signal exhibits dispersion and arranged to output for each set of electrogram signals a first array of probabilities each indicating whether a respective electrogram signal of the set of electrogram signals exhibits dispersion, a second evaluator comprising a convolutional neural network which receives a set of real-time electrograms signals and outputs a second array of probabilities that the input real-time electrogram signals exhibit dispersion, said convolutional neural network having been trained with a database of electrogram signals each labelled with a value indicating whether the respective electrogram signal exhibits dispersion, and a predictor which, based on the first array of probabilities and on the second array of probabilities determined for a given set of electrogram signals, returns a third array of probabilities, based at least in part on a weighted average of the values of the first array of probabilities and of the second array of probabilities.

This computer device is particularly interesting because it has allowed the Applicant to build patients' dispersion maps in real-time and to use these maps to reach an 88% termination rate of atrial fibrillation in those patients. This means that the Applicant's invention can be reliably used to assist practitioners in real-time to build dispersion maps and cure atrial fibrillation. The invention is as defined in the appended claims.

In various embodiments, the computer device may present one or more of the following features:
- the extractor is arranged to extract at least one timewise analysis feature in the group comprising a first cycle length estimation, a second cycle length estimation and the frequency within the Fast Fourier Transform of the electrogram signal which has the highest amplitude,
- the extractor is arranged to extract at least one morphological feature in the group comprising the Euclidian norm of the electrogram signal, and the integrated absolute derivative of the electrogram signal,
- the device is further arranged to divide a real-time electrogram signal into a series of electrogram signals shaving a chosen duration,
- the device is further arranged to provide a set of electrogram signals having the same chosen duration,
- the device is further arranged to divide a real-time electrogram signal into a series of electrogram signals having a chosen duration.
- the predictor is further arranged, when the absolute difference between a probability in the first array of probabilities and a probability in the second array of probabilities corresponding to the same electrogram signal exceeds a threshold, to use the probability of the first array of probabilities in the third array of probabilities, and
- the device is further arranged to determine a color associated to the values within the third array, the computer device further comprising a display arranged to output, for each electrode, the color associated to the probability determined for the corresponding electrogram signal in the third array of probabilities.

The invention also concerns a computer program comprising instructions for implementing the first evaluator, the second evaluator and the predictor, , a data storage medium having recorded thereon this computer program and a computer method of receiving real-time electrogram signals, executing the first evaluator, the second evaluator and the predictor according to the invention, and returning said third array of probabilities.

Other features and advantages of the invention will readily appear in the following description of the drawings, which show exemplary embodiments of the invention and on which:
- [Fig. 1] shows a general diagram on an embodiment of the computer device according to the invention,
- [Fig. 2] shows a generic view of a convolutional neural network used in the embodiment of Figure 1.

The drawings and the following description are comprised for the most part of positive and well-defined features. As a result, they are not only useful in understanding the invention, but they can also be used to contribute to its definition, should the need arise.

Persistent atrial fibrillation remains a therapeutic challenge. While an increasingly larger number of patients is eligible for catheter ablation, the optimal ablation strategy for persistent atrial fibrillation remains elusive. STAR-AF2 (Verma A, Jiang C-y, Betts TR et al. "Approaches to catheter ablation for persistent atrial fibrillation". New England Journal of Medicine 2015; 372:1812-1822) concluded that performing additional ablation lesions beyond pulmonary vein isolation (PVI) does not lead to improved long-term outcomes.

By contrast, some observational studies (see Jadidi AS, Lehrmann H, Keyl C et al. "Ablation of persistent atrial fibrillation targeting low-voltage areas with selective activation characteristics", Circulation: Arrhythmia and Electrophysiology 2016;9:e002962 and Seitz J, Bars C, Théodore G et al. "AF ablation guided by spatiotemporal electrogram dispersion without pulmonary vein isolation: a wholly patient-tailored approach", Journal of the American College of Cardiology 2017;69:303-321) have suggested that non-PVI lesions targeting regions harboring electrogram abnormalities are beneficial to patients with persistent atrial fibrillation.

In particular, the latter study showed the results of which supported the application of radiofrequency energy in atrial regions exhibiting the dispersion of multipolar electrograms. Other studies (Narayan SM, Krummen DE, Shivkumar K, Clopton P, Rappel W-J, Miller JM. "Treatment of atrial fibrillation by the ablation of localized sources: CONFIRM (Conventional Ablation for Atrial Fibrillation With or Without Focal Impulse and Rotor Modulation) trial", Journal of the American College of Cardiology 2012;60:628-636) instead performed advanced signal analysis and provided a mechanistic-based display to guide operators towards atrial fibrillation drivers.

Regardless of the electrogram abnormalities or analyses, there are large differences in experience and stage in the learning curve between centers attempting to implement similar electrogram-based mapping approaches.

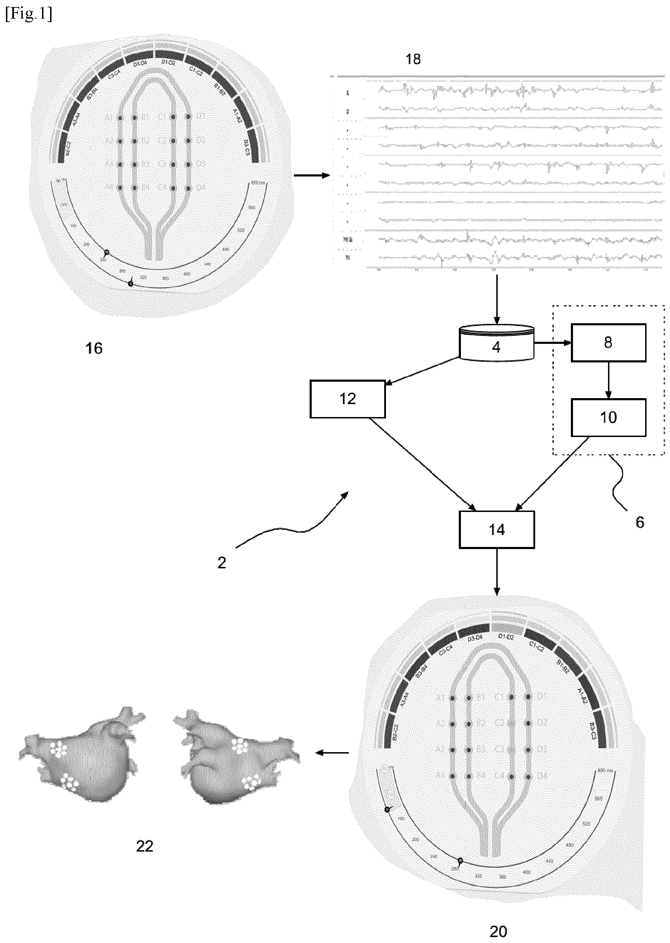

Figure 1 shows a general diagram on an embodiment of the computer device according to the invention.

Computer device 2 comprises a memory 4, a first evaluator 6 comprising an extractor 8 and a gradient boosting based machine learning module 10, a second evaluator 12 and a predictor 14.

From a usage perspective, an operator uses electrodes inserted in a patient's heart, which are represented on a display of the computer device 2 as exemplified with numeral reference 16. As apparent on Figure 1, there may be 16 electrodes of various type.

The result of the sensing by the electrodes is a set of real-time electrogram signals 18 which are fed to the memory 4. These electrogram signals are analyzed and the predictor 14 returns a modified view 20 of the electrodes which allows the practitioner to identify which of the electrodes have sensed a zone which may be associated with dispersion. In return, the practitioner may mark (or otherwise have the associated position returned in an automatic manner) the corresponding locations on a patient's heart, which leads to a map 22 of a heart with zones to be treated in view of atrial fibrillation represented as dots.

The memory 4 stores the real-time electrogram signals (for example after they have been digitalized and split at regular time intervals, such that all electrogram signals have the same length, e.g., 3 seconds) as well as a database of over 275000 electrograms signals labelled with a value indicating whether they exhibit dispersion, i.e., are associated with atrial fibrillation or not. The electrograms having identical duration may be consecutive or may overlap one another. Memory 4 may also store any transitory data which may be generated in the course of executing the invention. Memory 4 may also store the maps resulting from the operation of the predictor 14, possibly combined with practitioner made annotations as well as catheter geometry.

In the example described herein, the memory 4 may be realized in any way suitable, that is by means of a hard disk drive, a solid-state drive, a flash memory, a memory embedded in a processor, a distant storage accessible in the cloud, etc.

In the example described herein, the first evaluator 6 comprising the extractor 8 and the gradient boosting based machine learning module 10, the second evaluator 12 and the predictor 14 are computer programs which are executed on one or more processors. Such processors include any means known for performing automated calculus, such as CPUs, GPUs, CPUs and/or GPUs grids, remote calculus grids, specifically configured FPGAs, specifically configured ASICs, specialized chips such as SOCs or NOCs, AI specialized chips, etc.

In the example described herein, the first evaluator 6 is a machine learning module which is arranged to extract features from electrogram signals, and to run them through a gradient boost method in order to determine a dispersion probability.

The extractor 8 is arranged to determine set of features of each real-time electrogram signal, which the Applicant has identified as allowing to achieve excellent dispersion detection when applying the machine learning method. These features may be classified in two main feature categories.

The first category relates to the timewise analysis of the electrogram signals, i.e., features which are intrinsically related to the time domain or frequency domain analysis of the electrogram signals. In the example described herein, the extractor 8 may extract a first cycle length estimation, a second cycle length estimation, and/or the frequency within the Fast Fourier Transform of the electrogram signal which has the highest amplitude. In the latter case, the amplitude may also be retained as a feature.

The first cycle length estimation and second cycle length estimation may be determined as follows.

The real-time electrogram signal data are stored in a vector, each element of which corresponds to a sample thereof. Then, an autocorrelation parameter T is used to define two vectors of size T: a first vector comprising the first T samples of the vector, and a second vector comprising the last T samples of the vector.

In the example described here, the first estimator is calculated by measuring a normalized autocorrelation by taking the scalar product of the first vector and the second vector, divided by the product of the Euclidean norms of the first vector and the second vector. By varying T, a maximum and a minimum value of the first estimator is determined, and then the value selected for the first estimator is chosen as that for which the value of T provides the first local extremum for which the first estimator calculated with this value of T is greater than the difference between the maximum value of the first estimator subtracted by 0.3 times the difference between the maximum value and the minimum value of the first estimator. Alternatively, the first estimator can be determined differently, for example by changing the 0.3 coefficient, or by looking for a value of T that optimizes the estimator, empirically, exhaustively, or otherwise.

In the example described here, the second estimator is calculated by computing for each value of T the Euclidean norm squared by the difference between the first vector and the second vector, divided by the product of the sample with the largest absolute value in the first vector by the sample with the largest absolute value in the second vector. As with the first estimator, a maximum and a minimum value of the second estimator are determined, and then the value selected for the second estimator is chosen as the one for which the value of T provides the first local extremum for which the second estimator calculated with this value of T is smaller than the difference between the minimum value of the second estimator added by 0.2 times the difference between the maximum value and the minimum value of the second estimator. Alternatively, the first estimator can be determined differently, for example by changing the 0.2 coefficient, or by looking for a value of T that optimizes the estimator, empirically, exhaustively, or otherwise.

The second category relates to morphological analysis of the signal, i.e., analysis based on or related to mathematical morphology. In the example described herein, the extractor 8 may extract the Euclidian norm of the electrogram signal and the integrated absolute derivative of the electrogram signal.

By using all of the above referenced features, the gradient boosting based machine learning module 10 may use up to 6 distinct features for each real-time electrogram signal. Of course, in other embodiments, the extractor 8 may extract more features, and these features may be related to the combination or comparison of electrogram signals related to different electrodes by sharing an identical timecode. In some realizations, the Applicant has made use of up to 70 features in the gradient boosting based machine learning module 10.

The training of the gradient boosting based machine learning module 10 is made by using the 275000 electrograms signals labelled with a value indicating whether they exhibit dispersion. First the features are extracted for each of these electrogram signals, and then the gradient boosting based machine learning module 10 is trained in the example described herein using a logistic loss. After the training, the gradient boosting based machine learning module 10 can be used to receive set of features extracted from a set of electrograms as inputs and to return an array of values which each indicate the probability that a corresponding one of the electrogram signals in of the set of electrogram signals exhibits dispersion. Optionally the training data may also include catheter geometry information and/or 3D information relating thereto.

In the example described herein, the second evaluator 12 is a convolutional neural network which receives sets of electrogram signals, and outputs an array of values which each indicate the probability that a corresponding one of the electrogram signals in of the set of electrogram signals exhibits dispersion.

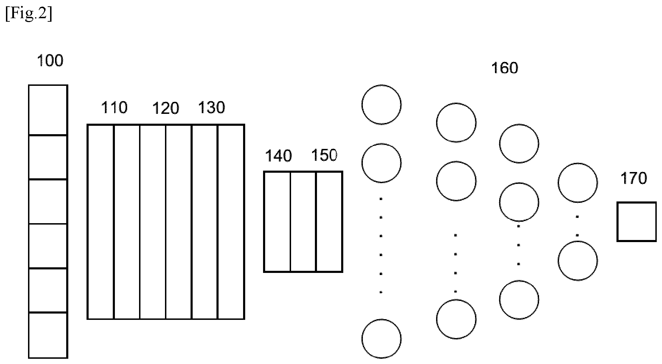

Figure 2 represents an exemplary structure for the convolutional neural network of second evaluator 12.

In the example described herein, the neural network is a convolutional neural network with 5 convolution layers. Thus, a set of electrogram signals 100 (comprising 16 electrograms for instance) is processed by a first convolution layer 110 which extracts 200 features, followed by a second convolution layer 120 which extracts 3000 features from layer 110, a third convolution layer 130 which extracts 20000 features from layer 120, a fourth convolution layer 140 which extracts 8000 features from a max pooling of layer 130, and a fifth convolution layer 150 which extracts 1000 features from layer 140.

The fifth convolution layer 150 is linked to a fully connected layer 160 of the neural network which comprises a chain of 2 layers of neurons comprising respectively 50 and 10 neurons. The fully connected layer 160 returns the array of probabilities 170 in the output layer.

The training of this convolutional neural network is made using by using the 275000 electrogram signals labelled with a value indicating whether they exhibit dispersion. The electrogram signals are used by groups of 16, each electrogram signal having its corresponding value of 0 or 1 indicating the presence of dispersion or not. The training is performed in the example described herein using an Adam optimizer and a binary cross entropy loss. Other optimizer and loss may be used, and the optimizer may be bypassed.

The predictor 14 is used to reconcile the predictions of the first evaluator 6 and those of the second evaluator 12. More precisely, in many cases, the second evaluator 12 is more robust against overfitting than the first evaluator 6, which is in turn usually more precise. For this reason, the predictor 14 may perform a weighted average of the values of the array of probabilities output respectively by the first evaluator 6 and the second evaluator 12. In one embodiment, the weights may be 0.7 for the first evaluator 6 and 0.3 for the second evaluator 12. Optionally, as convolutional neural networks are more prone to drift than gradient boosted machine learning methods, if the difference between the prediction by the first evaluator 6 and the second evaluator 12 is too great, the predictor 14 may opt to retain only the value of the first evaluator 6. The output of the predictor 14 is thus another array of probabilities that a given real-time electrogram signal exhibits dispersion.

The computer 14 may further determine colors based on the prediction array. More precisely, this determination can be based by using prediction arrays associated with consecutive timecodes. If it is determined that the atrial fibrillation of the patient is slow, two prediction arrays corresponding to consecutive timecodes can be averaged, and the values of this array be associated as follows: blue if the value is less than 0.35, orange if the value is comprised between 0.35 and 0.65, and else red. If. If it is determined that the atrial fibrillation of the patient is fast, four prediction arrays corresponding to consecutive timecodes can be averaged, and the values of this array be associated as follows: blue if the value is less than 0.5, orange if the value is comprised between 0.5 and 0.8, and else red. The determination of whether the atrial fibrillation is fast or slow is a practitioner decision, which he inputs in the device. This allows to provide a visual return on a display (as evidenced by reference numeral 20 on Figure 1), which may be used by the practitioner to tag automatically, manually or semi-manually the heart region, resulting in the dispersion areas identified by dots in reference numeral 22 of Figure 1.

While the above describes the use of two evaluators, any device or method using more than two evaluators which comprises the first evaluator and the second evaluator according to the invention shall fall under the scope of the following claims.

## Claims

1. A computer device for real-time analysis of electrograms, comprising a memory (4) arranged to receive real-time electrogram signals each originating from one of a plurality of electrodes, a first evaluator (6) comprising an extractor (8) and a gradient boosting based machine learning module (10), said extractor (8) being arranged to extract a set of features comprising at least one timewise analysis feature and at least one morphological feature from each electrogram signal within a set of real-time electrogram signals, and to feed the resulting sets of features to said gradient boosting based machine learning module (10) trained on data comprising sets of features labelled with a value indicating whether the associated electrogram signal exhibits dispersion and arranged to output for each set of electrogram signals a first array of probabilities each indicating whether a respective electrogram signal of the set of electrogram signals exhibits dispersion, a second evaluator (12) comprising a convolutional neural network which receives a set of real-time electrogram signals and outputs a second array of probabilities that the input real-time electrogram signals exhibit dispersion, said convolutional neural network having been trained with a database of electrogram signals each labelled with a value indicating whether the respective electrogram signal exhibits dispersion, and a predictor (14) which, based on the first array of probabilities and on the second array of probabilities determined for a given set of electrogram signals, returns a third array of probabilities, based at least in part on a weighted average of the values of the first array of probabilities and of the second array of probabilities.

2. Computer device according to claim 1, wherein the extractor (8) is arranged to extract at least one timewise analysis feature in the group comprising a first cycle length estimation, a second cycle length estimation and the frequency within the Fast Fourier Transform of the electrogram signal which has the highest amplitude.

3. Computer device according to claim 1 or 2, wherein the extractor (8) is arranged to extract at least one morphological feature in the group comprising the Euclidian norm of the electrogram signal, and the integrated absolute derivative of the electrogram signal.

4. Computer device according to one of the preceding claims, further arranged to divide a real-time electrogram signal into a series of electrogram signals having a chosen duration.

5. Computer device according to claim 4, further arranged to provide a set of electrogram signals having the same chosen duration.

6. Computer device according to one of the preceding claims, wherein the predictor (14) is further arranged, when the absolute difference between a probability in the first array of probabilities and probability in the second array of probabilities corresponding to the same electrogram signal exceeds a threshold, to use the probability of the first array of probabilities in the third array of probabilities.

7. Computer device according to one of the preceding claims, further arranged to determine a color associated to the values within the third array, the computer device further comprising a display arranged to output, for each electrode, the color associated to the probability determined for the corresponding electrogram signal in the third array of probabilities.

8. A computer implemented method comprising the steps executed by the computer device as defined in any of the preceding claims.

9. A computer program comprising instructions for implementing the method of claim 8 when said program is executed on a computer.

10. A data storage medium having recorded thereon the computer program of claim 9.

## Patentansprüche

1. Computervorrichtung zur Echtzeitanalyse von Elektrogrammen, umfassend einen Speicher (4), der so ausgelegt ist, dass er Echtzeit-Elektrogrammsignale empfängt, die jeweils von einer aus einer Vielzahl von Elektroden stammen, einen ersten Auswerter (6), der einen Extraktor (8) und ein auf Gradientenverstärkung basierendes Modul für maschinelles Lernen (10), wobei der Extraktor (8) so ausgelegt ist, dass er aus jedem Elektrogrammsignal innerhalb einer Gruppe von Elektrogrammsignalen eine Gruppe von Merkmalen extrahiert, die mindestens ein zeitliches Analysemerkmal und mindestens ein morphologisches Merkmal umfasst, und die resultierenden Merkmalssätze an das auf Gradientenverstärkung basierende maschinelle Lernmodul (10) weiterzuleiten, das auf Daten trainiert ist, die Merkmalssätze umfassen, die mit einem Wert gekennzeichnet sind, der angibt, ob das zugehörige Elektrogrammsignal eine Dispersion aufweist, und das so ausgelegt ist, dass es für jeden Satz von Elektrogrammsignalen ein erstes Array von Wahrscheinlichkeiten ausgibt, die jeweils angeben, ob ein jeweiliges Elektrogrammsignal des Satzes von Elektrogrammsignalen eine Dispersion aufweist, einen zweiten Evaluator (12), der ein konvolutionelles neuronales Netzwerk umfasst, das eine Gruppe von Echtzeit-Elektrogrammsignalen empfängt und ein zweites Array von Wahrscheinlichkeiten ausgibt, dass die eingegebenen Echtzeit-Elektrogrammsignale eine Dispersion aufweisen, wobei das konvolutionelle neuronale Netzwerk mit einer Datenbank von Elektrogrammsignalen trainiert wurde, die jeweils mit einem Wert gekennzeichnet sind, der angibt, ob das jeweilige Elektrogrammsignal eine Dispersion aufweist, und einen Prädiktor (14), der auf der Grundlage des ersten Wahrscheinlichkeitsarrays und des zweiten Wahrscheinlichkeitsarrays, die für eine gegebene Gruppe von Elektrogrammsignalen bestimmt wurden, ein drittes Wahrscheinlichkeitsarray zurückgibt, das zumindest teilweise auf einem gewichteten Durchschnitt der Werte des ersten Wahrscheinlichkeitsarrays und des zweiten Wahrscheinlichkeitsarrays basiert.

2. Computervorrichtung nach Anspruch 1, wobei der Extraktor (8) so ausgelegt ist, dass er mindestens ein zeitliches Analysemerkmal aus der Gruppe extrahiert, die eine erste Zyklusschätzungslänge, eine zweite Zyklusschätzungslänge und die Frequenz innerhalb der Fast-Fourier-Transformation des Elektrogrammsignals mit der höchsten Amplitude umfasst.

3. Computervorrichtung nach Anspruch 1 oder 2, wobei der Extraktor (8) so ausgelegt ist, dass er mindestens ein morphologisches Merkmal aus der Gruppe extrahiert, die die euklidische Norm des Elektrogrammsignals und die integrierte absolute Ableitung des Elektrogrammsignals umfasst.

4. Computervorrichtung nach einem der vorstehenden Ansprüche, die ferner dazu ausgelegt ist, ein Echtzeit-Elektrogrammsignal in eine Reihe von Elektrogrammsignalen mit einer gewählten Dauer zu unterteilen.

5. Computervorrichtung nach Anspruch 4, die ferner dazu ausgelegt ist, einen Satz von Elektrogrammsignalen mit derselben gewählten Dauer bereitzustellen.

6. Computervorrichtung nach einem der vorstehenden Ansprüche, wobei der Prädiktor (14) ferner so ausgelegt ist, dass er, wenn die absolute Differenz zwischen einer Wahrscheinlichkeit im ersten Wahrscheinlichkeitsarray und einer demselben Elektrogrammsignal entsprechenden Wahrscheinlichkeit im zweiten Wahrscheinlichkeitsarray einen Schwellenwert überschreitet, die Wahrscheinlichkeit des ersten Wahrscheinlichkeitsarrays im dritten Wahrscheinlichkeitsarray verwendet.

7. Computervorrichtung nach einem der vorstehenden Ansprüche, die ferner dazu ausgelegt ist, eine den Werten innerhalb des dritten Arrays zugeordnete Farbe zu bestimmen, wobei die Computervorrichtung ferner eine Anzeige umfasst, die dazu ausgelegt ist, für jede Elektrode die Farbe auszugeben, die der für das entsprechende Elektrogrammsignal im dritten Wahrscheinlichkeitsarray bestimmten Wahrscheinlichkeit zugeordnet ist.

8. Ein Computerprogramm, das die von der Computervorrichtung ausgeführten Schritte gemäß einem der vorstehenden Ansprüche umfasst.

9. Ein Computerprogramm, das Anweisungen zur Implementierung des Verfahrens nach Anspruch 8 umfasst, wenn das Programm auf einem Computer ausgeführt wird.

10. Ein Datenspeichermedium, auf dem das Computerprogramm nach Anspruch 9 aufgezeichnet ist.

## Revendications

1. Dispositif informatique destiné à l'analyse en temps réel d'électrogrammes, comprenant une mémoire (4) agencée pour recevoir des signaux d'électrogrammes en temps réel provenant chacun d'une parmi une pluralité d'électrodes, un premier évaluateur (6) comprenant un extracteur (8) et un module d'apprentissage automatique basé sur le gradient boosting (10), ledit extracteur (8) étant agencé pour extraire un ensemble de caractéristiques comprenant au moins une caractéristique d'analyse temporelle et au moins une caractéristique morphologique de chaque signal d'électrogramme au sein d'un ensemble de signaux d'électrogramme, et pour transmettre les ensembles de caractéristiques résultants audit module d'apprentissage automatique basé sur le gradient boosting (10) entrainé sur des données comprenant des ensembles de caractéristiques étiquetées avec une valeur indiquant si le signal d'électrogramme associé présente une dispersion et agencé pour produire pour chaque ensemble de signaux d'électrogramme un premier tableau de probabilités indiquant chacune si un signal d'électrogramme respectif de l'ensemble de signaux d'électrogramme présente une dispersion, un deuxième évaluateur (12) comprenant un réseau neuronal convolutif qui reçoit un ensemble de signaux d'électrogrammes en temps réel et produit un deuxième tableau de probabilités que les signaux d'électrogramme en temps réel d'entrée présentent une dispersion, ledit réseau neuronal convolutif ayant été entraîné avec une base de données de signaux d'électrogramme, chacun étant étiqueté avec une valeur indiquant si le signal d'électrogramme respectif présente une dispersion, et un prédicteur (14) qui, sur la base du premier tableau de probabilités et du deuxième tableau de probabilités déterminés pour un ensemble donné de signaux d'électrogramme, renvoie un troisième tableau de probabilités, basé au moins en partie sur une moyenne pondérée des valeurs du premier tableau de probabilités et du deuxième tableau de probabilités.

2. Dispositif informatique selon la revendication 1, dans lequel l'extracteur (8) est agencé pour extraire au moins une caractéristique d'analyse temporelle dans le groupe comprenant une estimation de longueur d'un premier cycle, une estimation de longueur d'un deuxième cycle et la fréquence dans la transformée de Fourier rapide du signal d'électrogramme, qui présente l'amplitude la plus élevée.

3. Dispositif informatique selon la revendication 1 ou 2, dans lequel l'extracteur (8) est agencé pour extraire au moins une caractéristique morphologique parmi le groupe comprenant la norme euclidienne du signal d'électrogramme et la dérivée absolue intégrée du signal d'électrogramme.

4. Dispositif informatique selon l'une des revendications précédentes, agencé en outre pour diviser un signal d'électrogramme en temps réel en une série de signaux d'électrogramme ayant une durée choisie.

5. Dispositif informatique selon la revendication 4, agencé en outre pour fournir un ensemble de signaux d'électrogramme ayant la même durée choisie.

6. Dispositif informatique selon l'une des revendications précédentes, dans lequel le prédicteur (14) est en outre agencé, lorsque la différence absolue entre une probabilité du premier tableau de probabilités et une probabilité du deuxième tableau de probabilités correspondant au même signal d'électrogramme dépasse un seuil, pour utiliser la probabilité du premier tableau de probabilités dans le troisième tableau de probabilités.

7. Dispositif informatique selon l'une des revendications précédentes, agencé en outre pour déterminer une couleur associée aux valeurs du troisième tableau, le dispositif informatique comprenant en outre un écran agencé pour afficher, pour chaque électrode, la couleur associée à la probabilité déterminée pour le signal d'électrogramme correspondant dans le troisième tableau de probabilités.

8. Programme informatique comprenant les étapes exécutées par le dispositif informatique tel que défini dans l'une quelconque des revendications précédentes.

9. Programme informatique comprenant des instructions pour mettre en œuvre le procédé de la revendication 8 lorsque ledit programme est exécuté sur un ordinateur.

10. Support de stockage de données sur lequel est enregistré le programme informatique de la revendication 9.
